# DEMANDE DE BREVET EUROPEEN

(11) **EP 1 671 670 A1**
(43) Date de publication de la demande: **21.06.2006**
(21) Numéro de dépôt: 04447276.9
(22) Date de dépôt: 14.12.2004
(51) Int. Cl.: A61N 1/32

(54) **Appareil pour l'electro-inhibition des muscles de la face**

(71) Demandeur: STX Sprl, 4020 Liege (BE)
(72) Inventeur: Rigaux, Pierre, 4020 Liège (BE)
(74) Mandataire: Van Malderen, Michel

(57) **Abrégé**

La présente invention se rapporte à un dispositif autonome (1) pour réaliser une inhibition électrique de muscles de la face, comprenant deux électrodes de contact (2), un circuit électronique (3) pour la création d'impulsions électriques basse tension au niveau desdites électrodes (2), une alimentation électrique DC (4) et des moyens de fixation et blocage (7) des éléments précités sur la tête d'un patient, caractérisé en ce que les électrodes (2) sont configurées pour être disposées de chaque côté de la partie supérieure du nez et de manière à faire passer un courant électrique au travers du muscle pyramidal du nez sous forme d'impulsions de fréquence supérieure à 100 Hz et de préférence supérieure à 150 Hz, applicables pendant une durée d'au moins une minute, et de préférence comprise entre 1 et 5 minutes.

## Description

### Objet de l'invention

La présente invention se rapporte à un appareil d'inhibition électrique ou "électro-inhibition" permettant d'obtenir le relâchement de certains muscles de la partie supérieure du visage.

### Arrière-plan technologique et état de la technique

Les rides peuvent être dues à une multitude de facteurs comme l'âge, les dommages causés par le soleil, etc., mais elles sont surtout dues aux mimiques, c'est-à-dire aux contractions musculaires répétées (expression de joie, de colère, de tristesse, de peur, d'étonnement, etc.). On parle donc de "rides d'expression". Ces rides qui apparaissent principalement dans la moitié supérieure du visage sont dues aux interactions entre la peau et les muscles sous-jacents. On connaît notamment les rides verticales du front, les rides du coin des yeux appelées "pattes d'oie", les rides horizontales du front, etc.

Ainsi, les rides du front, de même que la plupart des céphalées chroniques, communément appelés "maux de tête" ou "migraines", ont pour cause une tension prolongée induisant une contracture chronique soit de l'un des trois muscles de la partie supérieure de la face qui sont le muscle frontal (*frontalis*), le double muscle sourcilier (*corrugator*) et le muscle pyramidal du nez (*procerus*), soit encore de plusieurs d'entre eux en même temps.

Le muscle pyramidal est un petit faisceau musculaire charnu situé sur la partie supérieure du dos du nez et entre les yeux. Il s'étend entre le fascia recouvrant la partie inférieure de l'os propre du nez et la face profonde de la peau de la région inter-sourcilière. Il provoque une attraction des sourcils vers le bas et la ligne médiane, en créant des replis sur la racine du nez. Il intervient dans le froncement des sourcils, l'expression de la concentration ou tout simplement pour réduire l'éblouissement en cas de lumière vive.

Le muscle frontal est un muscle plat situé sous la peau du front. Il naît du bord antérieur de l'aponévrose épicrânienne, ses fibres charnues descendent et s'attachent à la face profonde de la peau des régions sourcilière et inter-sourcilière. Il élève la peau des sourcils et produit les rides et replis horizontaux du front.

Les deux muscles sourciliers sont étendus de part et d'autre de la glabelle le long de la partie interne de l'arcade sourcilière. Ils naissent de l'extrémité interne de l'arcade sourcilière et se dirigent en dehors le long de l'arcade pour se terminer à la face profonde de la peau des sourcils. Ils attirent les sourcils vers la ligne médiane et sont responsable des rides et replis cutanés verticaux de la glabelle.

L'obtention d'un relâchement de ces muscles permettrait donc de lutter contre les céphalées et les rides de tension. De plus, un relâchement de ces trois muscles de la partie supérieure du visage est susceptible de permettre à l'activité des muscles de la zone occipitale de prédominer. Ces derniers sont le muscle occipital (*occipitalis*) et les deux muscles auriculaires postérieurs (*auricularis posterior*).

Ce changement d'équilibre entre muscles de la face et muscles de l'occiput à l'avantage de ces derniers est susceptible de produire un étirement de la peau de la face, avec effet de lifting physiologique, qui améliore l'apparence du visage.

On connaît actuellement différents traitements de correction des rides disgracieuses telles que rides du front, de la glabelle ou pattes d'oie, apparaissant sur les peaux vieillissantes.

Certains fabricants de crèmes de soins préconisent une gestuelle de massage appropriée pour décontracter les muscles faciaux. Cependant, on peut douter de l'efficacité d'un tel traitement, surtout lorsque les rides d'expression sont déjà bien marquées.

Il existe également des traitements d'injection de produits à action temporaire tel que l'acide hyaluronique ou la toxine botulinique de type A, dérivée de la bactérie *Clostridium* botulinum (connue sous le nom commercial Botox® ). Le Botox® est injecté en doses faibles dans un muscle spécifique qui devient paralysé et ne peut plus dès lors se contracter. Ces produits ont une durée d'action de quelques mois.

On injecte également des implants permanents à base de microbilles en plastique ou des implants tubulaires en PTFE, ainsi que du collagène ou de la silicone. La longévité des implants est de plusieurs années. Il s'agit d'une chirurgie éventuellement réversible.

Ces techniques d'injection présentent toutefois un certain nombre d'effets secondaires tels que risque de développer une allergie, oedème après injection, risque d'infection, rougeurs, ecchymoses, sensations de gêne, etc.

De plus, les injections de Botox® se retrouvent dans la circulation générale du patient. Un effet secondaire spécifique est notamment une observation de paralysie indésirable des muscles de la déglutition (dysphagie) .

Par ailleurs, l'électro-stimulation musculaire est bien connue. Ainsi, par exemple, le brevet américain US-A-4,957,480 décrit une méthode de tonification des muscles et tissus du visage par stimulation des nerfs moteurs et contraction consécutive des muscles de la face, par application de courants galvaniques d'amplitude, de fréquence et de polarité prédéterminés au travers d'électrodes humidifiées au moyen d'une solution liquide de particules chargées positivement et négativement, en vue de son introduction dans les tissus pour nourrir les muscles et les tissus du visage environnants. On sait, à partir de ce document, qu'il est possible de cette manière, par un positionnement particulier des électrodes directement sur le muscle, d'obtenir une fatigue, et partant une relaxation, par exemple du muscle sourcilier ou du muscle frontal. Les courants sont compris entre 300 et 640 µA avec une fréquence comprise entre 30 et 99 Hz, avec une polarisation alternée et une durée d'application comprise entre 1 et 4 secondes, voire même 10 secondes. Les paramètres électriques utilisés génèrent une fatigue de travail, semblable à la fatigue volontaire du muscle, qui ne produit qu'une relaxation peu marquée sinon nulle. Il s'agit du cadre classique de l'électro-stimulation avec des fréquences inférieures à 100 Hz pour produire des contractions courtes.

Le brevet américain US-A-3,709,228 décrit un appareil d'électro-stimulation des nerfs, et partant des muscles, de la face d'un utilisateur, comprenant un support reposant sur le nez et les oreilles et portant des bras déformables pouvant s'étendre dans des directions opposées et porteurs à leur extrémité d'électrodes de contact avec la peau.

### Buts de l'invention

La présente invention vise à fournir un appareil permettant d'obtenir, par l'action d'un courant électrique, un relâchement d'un ou plusieurs muscles de la partie supérieure du visage, en particulier le muscle frontal (*frontalis*), les deux muscles sourciliers (*corrugator*) et le muscle pyramidal du nez (*procerus*).

L'invention vise en particulier à proposer une méthode de correction des rides et de soin des céphalées chroniques, qui soit simple, non invasive et dépourvue d'effets secondaires.

### Principaux éléments caractéristiques de l'invention

La présente invention a pour objet un dispositif autonome pour réaliser une inhibition électrique de certains muscles de la face, comprenant deux électrodes de contact, un circuit électronique pour la création d'impulsions électriques basse tension au niveau desdites électrodes, une alimentation électrique DC et des moyens de fixation et blocage des éléments précités sur la tête d'un patient, caractérisé en ce que les électrodes sont configurées pour être disposées de chaque côté de la partie supérieure du nez et de manière à faire passer un courant électrique au travers du muscle pyramidal du nez sous forme d'impulsions de fréquence supérieure à 100 Hz et de préférence supérieure à 150 Hz, applicables pendant une durée d'au moins une minute, et de préférence comprise entre 1 et 5 minutes.

Selon l'invention, les électrodes ont une forme rectangulaire et sont montées essentiellement parallèles avec le grand côté du rectangle placé verticalement et séparées l'une de l'autre par une distance comprise entre 3 et 15 mm.

De préférence, les impulsions ont une largeur comprise entre 30 et 100 µs et une intensité comprise entre 0 et 30 mA.

Selon une première modalité d'exécution préférée, les moyens de fixation et blocage sont du type monture de lunettes avec un appui à l'avant sur le nez et deux branches qui se positionnent derrière les oreilles.

Selon une deuxième modalité d'exécution préférée, les moyens de fixation et blocage comprennent un serre-tête destiné à se positionner au niveau du front.

Selon encore une autre modalité d'exécution préférée, les moyens de fixation et blocage comprennent un montage avec surface autocollante pour le positionnement au niveau de la glabelle.

Selon encore une autre modalité d'exécution préférée, les moyens de fixation et blocage comprennent une arcade de soutien s'appuyant sur la ligne médiane du crâne.

Selon encore une autre modalité d'exécution préférée, les moyens de fixation et blocage comprennent un casque en matière légère.

De préférence, les moyens de blocage sont configurés pour que, en position verrouillée, respectivement en position déverrouillée, le système fait également office d'interrupteur fermé, respectivement ouvert, dudit circuit électronique.

Un deuxième objet de l'invention concerne l'utilisation du dispositif d'électro-inhibition musculaire décrit ci-dessus, pour le relâchement du muscle pyramidal du nez, de son muscle antagoniste, le muscle frontal et du double muscle sourcilier.

### Brève description des figures

La figure 1 représente schématiquement une vue avant du dispositif d'électro-inhibition selon la présente invention, placé sur la tête d'un patient.

La figure 2 représente une vue arrière du dipositif de la figure 1.

La figure 3 représente une vue du dessus pour le dispositif de la figure 1.

La figure 4 représente une vue en coupe et du dessus de la partie avant du dispositif de la figure 1.

### Description d'une forme d'exécution préférée de l'invention

### Méthode

Le principe innovant à la base de l'invention est de produire une inhibition des trois muscles précités dans la partie supérieure de la face au moyen de l'application d'un courant électrique sur un seul de ces muscles, à savoir le pyramidal. Il s'agit donc d'une méthode visant l'effet inverse de celui recherché dans l'électro-stimulation, puisque le but recherché est le relâchement des muscles et non leur contraction.

### Description de la technique

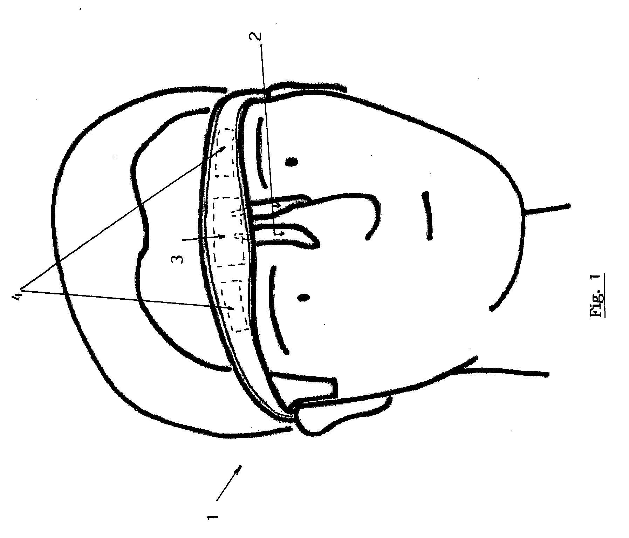

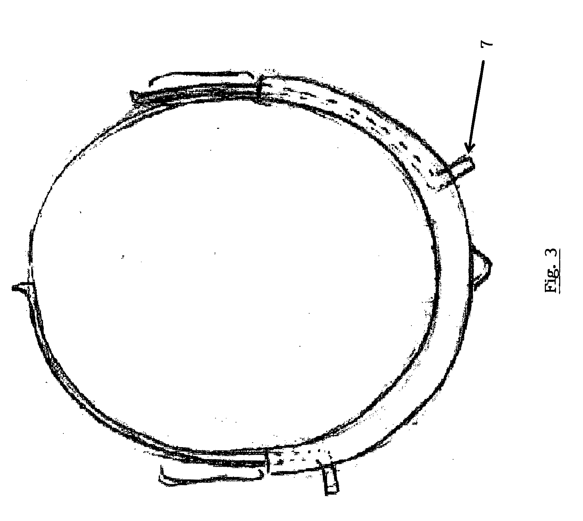

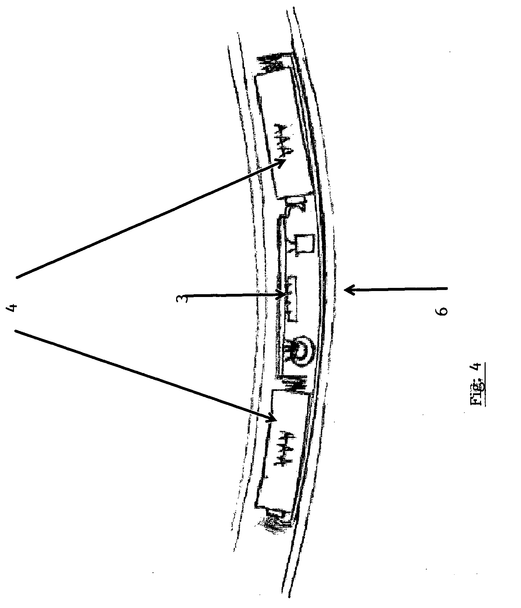

Selon l'invention représentée sur les figures 1 à 4, deux petites électrodes 2 sont positionnées parallèlement l'une à l'autre au niveau de l'espace inter-sourcilier appelé glabelle (*glabella*), de façon à faire passer le courant électrique d'inhibition au travers du muscle pyramidal du nez. Les électrodes 2 sont supportées par un serre-tête 1 .

Ces électrodes 2 ont essentiellement une forme rectangulaire dont la taille est plus ou moins de 25 mm sur 5 mm et sont seulement écartées l'une de l'autre par une distance de 3 à 15 mm.

L'avantage de ce placement particulier des électrodes est double :
- simplicité : avec seulement deux électrodes et un seul canal de stimulation on obtient l'effet de relaxation sur les trois muscles-cibles en même temps grâce aux mécanismes physiologiques décrits plus loin (fatigue électrique, réflexe d'inhibition et excitation des afférences tendineuses) ;
- confort : le très faible écartement entre les deux électrodes (3 à 15 mm) permet de limiter les sensations désagréables en réduisant le plus possible le volume des tissus soumis au courant électrique ^{(1,2)} . En effet, ce faible écartement des électrodes a pour double conséquence, d'une part, qu'une très petite surface de peau est soumise au courant électrique et, d'autre part, une très faible pénétration du courant en profondeur, celui-ci circulant donc préférentiellement dans les couches superficielles de l'épiderme sans exciter les terminaisons nerveuses du périoste, très sensibles à la douleur.

Le courant appliqué aux électrodes 2 est constitué d'impulsions électriques connues pour être capables de déclencher des potentiels d'action (PA) au niveau des nerfs moteurs. Il s'agit d'impulsions rectangulaires d'une largeur comprise entre 30 et 100 µs et d'une intensité allant de 0 à 30 mA (3). L'utilisation de ce type d'impulsions conduit à une moindre excitation des fibres de la douleur que l'utilisation d'impulsions plus longues. Mais tout autre type d'impulsion suffisante pour déclencher un PA sur des motoneurones pourrait être employé.

Une caractéristique essentielle de l'invention est que le courant appliqué utilise avantageusement des impulsions présentant une fréquence supérieure à 100 Hz, ce qui génère une fatigue électrique très rapide du muscle pyramidal ^{(4,5)}. Il se produit grâce à cette excitation continue des nerfs moteurs une extinction rapide de l'activité électrique des fibres musculaires innervées par les nerfs moteurs excités ⁽⁶⁾. Cette fréquence d'impulsion étant maintenue constante, en quelques secondes, le muscle pyramidal devient inexcitable, perd son tonus et ainsi se relâche totalement ⁽⁷⁾.

L'excitation des motoneurones du muscle pyramidal produit également un relâchement de son muscle antagoniste -le muscle frontal- via le réflexe d'inhibition réciproque ⁽⁸⁾. En effet, les impulsions électriques au niveau d'un muscle excitent non seulement les motoneurones de ce muscle mais aussi les fibres afférentes proprioceptives de ce muscle. L'excitation de ces dernières inhibe les motoneurones du muscle antagoniste ^{(9,10)}*.*

De plus, les électrodes 2 étant situées au niveau des tendons internes des muscles sourciliers, le courant excite les afférences nerveuses de ces tendons, c'est-à-dire celles qui quittent les corpuscules tendineux de Golgi. Ces afférences tendineuses étant inhibitrices des motoneurones de leur muscle, à savoir le double sourcilier, leur excitation par les impulsions électriques produit un relâchement du muscle ^{(11,12)}.

Selon l'invention, les fréquences électriques sont plus élevées (au moins 100 ou 150 Hz) et la durée d'application beaucoup plus longue (typiquement supérieure à 60 s), que dans le brevet US-A-4,957,480. Il se produit ainsi, non pas une fatigue de travail, mais une fatigue dite électrique (^{4,6}) . L'activité électrique, caractérisée par les potentiels de repos et d'action, sur les fibres musculaires disparaît par accumulation d'ions potassium (K⁺) en extra-cellulaire. Le couplage électromécanique de l'activité musculaire est rompu par la disparition du potentiel électrique des fibres. Ceci a pour conséquence une suspension totale de l'activité mécanique du muscle et donc une relaxation complète, le muscle étant en état réversible de sidération électromécanique.

### Le dispositif

Tout le système permettant l'électro-inhibition est inclus dans un dispositif unique 1, représenté sur les figures 1 à 4. Selon une première forme d'exécution préférée, celui-ci est conçu de façon similaire à une monture de lunettes qui repose sur le nez à l'avant et sur les oreilles à l'arrière grâce à deux branches adéquates. Le circuit électronique 3 et l'alimentation 4 sous forme de batteries sont placés dans une partie frontale creuse du dispositif, au niveau de trappes 5. Les électrodes sur la face interne et médiane de la partie frontale 6 sont configurées pour se positionner correctement dans l'espace inter-sourcilier lorsque le dispositif est mis en place sur un sujet.

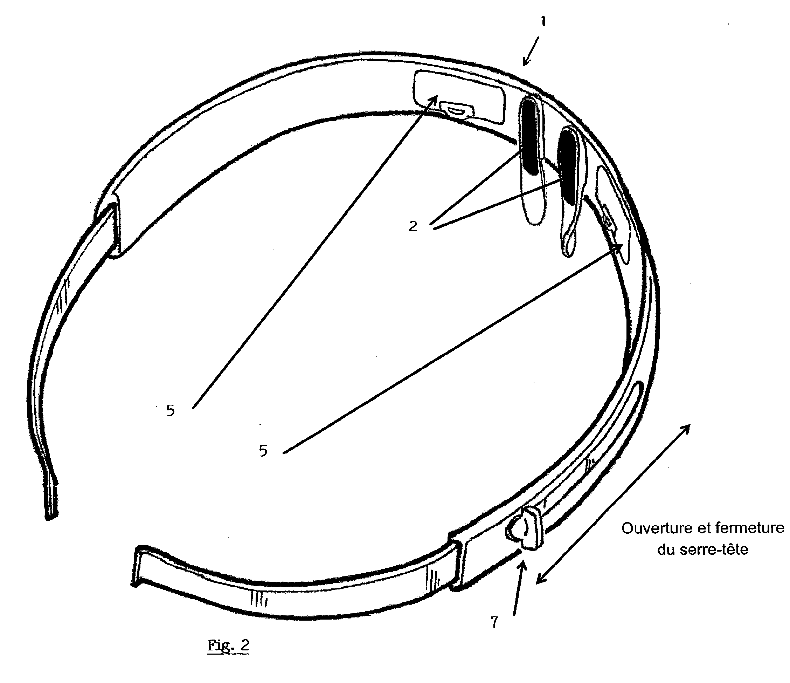

Selon la présente invention, plusieurs autres formes d'exécution avantageuses de ce dispositif sont possibles, telles que :
- montage sur un serre-tête, comme représenté sur la figure 2. Dans ce cas, on peut par exemple prévoir des moyens de blocage et fixation 7, qui peuvent servir également d'interrupteur enclenchant l'alimentation électrique, une fois le serre-tête en place sur le sujet ;
- montage sur une surface autocollante venant se placer au niveau de la glabelle ;
- arcade de soutien du dispositif s'appuyant sur la ligne médiane du crâne ;
- petit casque, etc.

### Avantages

Outre les avantages de simplicité et de confort déjà mentionnés ci-dessus, le dispositif de l'invention présente encore l'avantage d'être de réalisation peu coûteuse, d'utilisation très facile et brève (typiquement moins de 5 minutes), notamment au domicile de l'utilisateur et de ne pas induire les effets secondaires que l'on retrouve dans les techniques à injection (allergie, rougeurs, oedèmes, présence d'un implant artificiel, etc.).

### Références bibliographiques

⁽¹⁾ J. P. REILLY, *Impedance and current distribution,* Applied Bioelectricity, 2^{nd} Ed., Hardback, Springer-Verlag, New York, 1998, pp. 12-45.
⁽²⁾ L.A. GEDDES and L.E. BAKER, *Electrodes*, Principles of Applied Biomedical Instrumentation, 3^{rd} Ed., John Wiley & Sons, 1989, pp. 315-387.
⁽³⁾ L.A. GEDDES and L.E. BAKER, *Stimulators and Stimulation,* Principles of Applied Biomedical Instrumentation, 3rd Ed., John Wiley & Sons, 1989, pp. 453-469.
⁽⁴⁾ D.A. JONES and B. BIGLAND-RITCHIE, *Excitation Frequency and Muscle Fatigue : Mechanical Responses during Voluntary and Stimulated Contractions,* Experimental Neurology 64, pp. 401-413 (1979).
⁽⁵⁾ D.A. JONES, *Muscle Fatigue due to Changes beyond the Neuromuscular Junction,* Human Muscle Fatigue : Physiological Mechanisms, Pitman Medical London, 1981 (Ciba Foundation Symposium 82), pp. 178-196.
⁽⁶⁾ B. BIGLAND-RITCHIE, *EMG and Fatigue of Human Voluntary and Stimulated Contractions,* Human Muscle Fatigue : Physiological Mechanisms, Pitman Medical London 1981 (Ciba Foundation symposium 82), pp. 130-156.
(⁷) D.A. JONES, *High and Low-Frequency Fatigue Revisited,* Acta Physiol. Scand. 156, 265-270 (1996).
⁽⁸⁾ A.C. GUYTON, *Reciprocal Innervation,* Textbook of Medical Physiology, 5th Edition, Sanders Company, 1976, pp. 626-27.
⁽⁹⁾ J.B. WAL, *Modulation of Spasticity : Prolonged Suppression of a Spinal Reflex by Electrical Stimulation,* Science 216 : 203-204, 1982.
⁽¹⁰⁾ M.G. LEVIN, M. KNOTT and H. KABAT, *Relaxation of Spasticity by Electrical Stimulation* of *Antagonist Muscles,* Arch. Phys. Med. 33 : 668-673, 1952.
⁽¹¹⁾ A.C. GUYTON, *Tendon Reflex,* Textbook of Medical Physiology, 5th edition, Sanders Company, 1976, pp. 623-24.
⁽¹²⁾ J. HOUK and E. HENNEMAN, *Responses of Golgi tendon organs to active contractions of the soleus muscle of the cat,* J. Neurophysiol. 30 : 466, 1967.

## Revendications

1. Dispositif autonome (1) pour réaliser une inhibition électrique de muscles de la face, comprenant deux électrodes de contact (2), un circuit électronique (3) pour la création d'impulsions électriques basse tension au niveau desdites électrodes (2), une alimentation électrique DC (4) et des moyens de fixation et blocage (7) des éléments précités sur la tête d'un patient, **caractérisé en ce que** les électrodes (2) sont configurées pour être disposées de chaque côté de la partie supérieure du nez et de manière à faire passer un courant électrique au travers du muscle pyramidal du nez sous forme d'impulsions de fréquence supérieure à 100 Hz et de préférence supérieure à 150 Hz, applicables pendant une durée d'au moins une minute, et de préférence comprise entre 1 et 5 minutes.

2. Dispositif selon la revendication 1, **caractérisé en ce que** les électrodes (2) ont une forme rectangulaire et sont montées essentiellement parallèles avec le grand côté du rectangle placé verticalement et séparées l'une de l'autre par une distance comprise entre 3 et 15 mm.

3. Dispositif selon la revendication 2, **caractérisé en ce que** les impulsions ont une largeur comprise entre 30 et 100 µs et une intensité comprise entre 0 et 30 mA.

4. Dispositif selon l'une quelconque des revendications 1 à 3, **caractérisé en ce que** les moyens de fixation et blocage (7) sont du type monture de lunettes avec un appui à l'avant sur le nez et deux branches qui se positionnent derrière les oreilles.

5. Dispositif selon l'une quelconque des revendications 1 à 3, **caractérisé en ce que** les moyens de fixation et blocage (7) comprennent un serre-tête destiné à se positionner au niveau du front.

6. Dispositif selon l'une quelconque des revendications 1 à 3, **caractérisé en ce que** les moyens de fixation et blocage (7) comprennent un montage avec surface autocollante pour le positionnement au niveau de la glabelle.

7. Dispositif selon l'une quelconque des revendications 1 à 3, **caractérisé en ce que** les moyens de fixation et blocage (7) comprennent une arcade de soutien s'appuyant sur la ligne médiane du crâne.

8. Dispositif selon l'une quelconque des revendications 1 à 3, **caractérisé en ce que** les moyens de fixation et blocage (7) comprennent un casque en matière légère.

9. Dispositif selon l'une quelconque des revendications 4 à 8, **caractérisé en ce que** les moyens de blocage sont configurés pour que, en position verrouillée, respectivement en position déverrouillée, le système fait également office d'interrupteur fermé, respectivement ouvert, dudit circuit électronique (3).

10. Utilisation du dispositif d'électro-inhibition musculaire selon l'une quelconque des revendications précédentes, pour le relâchement du muscle pyramidal du nez, de son muscle antagoniste, le muscle frontal et du double muscle sourcilier.
